(19) Europäisches Patentamt
European Patent Office
Office européen des brevets

(11) Veröffentlichungsnummer: **0 153 657 A1**

(12) # EUROPÄISCHE PATENTANMELDUNG

(21) Anmeldenummer: 85101520.6

(22) Anmeldetag: 13.02.85

(51) Int. Cl.⁴: **C 07 D 249/08**
C 07 D 401/06, C 07 D 401/12
C 07 D 403/06, C 07 D 405/12
A 01 N 43/653

(30) Priorität: 25.02.84 DE 3406908

(43) Veröffentlichungstag der Anmeldung: 04.09.85 Patentblatt 85/36

(84) Benannte Vertragsstaaten: CH DE FR GB IT LI

(71) Anmelder: HOECHST AKTIENGESELLSCHAFT
Postfach 80 03 20
D-6230 Frankfurt am Main 80(DE)

(72) Erfinder: Ehrhardt, Heinz, Dr.
Bergstrasse 21
D-8901 Rehling(DE)

(72) Erfinder: Mildenberger, Hilmar, Dr.
Fasanenstrasse 24
D-6233 Kelkheim (Taunus)(DE)

(72) Erfinder: Handte, Reinhard, Dr.
Theilweg 23
D-8901 Gablingen(DE)

(72) Erfinder: Sachse, Burkhard, Dr.
An der Ziegelei 30
D-6233 Kelkheim (Taunus)(DE)

(72) Erfinder: Hartz, Peter, Dr.
An der Ziegelei 28
D-6233 Kelkheim (Taunus)(DE)

(72) Erfinder: Bürstell, Helmut, Dr.
Am Hohlacker 65
D-6000 Frankfurt am Main 50(DE)

(54) **Neue Triazolylalkylderivate, Verfahren zu ihrer Herstellung sowie ihre Verwendung als Pflanzenbehandlungsmittel.**

(57) Verbindungen der Formel (I),

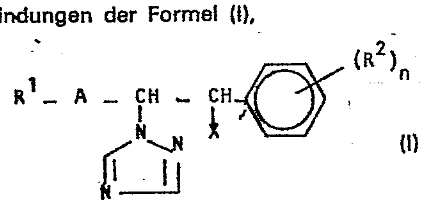

(I)

worin A = eine Carbonylgruppe oder die Gruppierung $-CH(OH)-$; $R^1$ = (subst.) Phenyl, (subst.) Alkyl, (subst. Cycloalkyl oder Bicycloalkyl; $R^2$ = Halogen, (subst.) Alkyl, Cycloalkyl, (subst.) Alkoxy, Alkylthio, Alkenyloxy, Dialkylamino, Nitro, Cyano, Hydroxy, Alkoxycarbonyl, Phenyl, Phenoxy oder zwei Reste $R^2$ eine C-Kette; n = 0, 1, 2 oder 3; X = $-S(O)_mR^5$ (mit m = 0, 1 oder 2), subst.) Amino, (subst.) Piperidino, (subst.) Morpholino, $CHR^8R^9$, (subst.)Triazolyl, (subst.) Pyrazolyl, (subst.) Pyrimidinyl; $R^5$ = (subst.) Alkyl, Alkenyl, Cycloalkyl, Aminoalkyl, Dialkylaminoalkyl, Dicarboxyalkyl, $R^{11}-O-CO-$(amino)alkyl, $R^{12}-S-CO-$alkyl; (subst.) Amino$-CO-$Alkyl, Alkanoyl, (subst.) Benzoyl, (subst.) Phenyl oder (subst.) Benzyl, Furfuryl oder einen O, S oder N-Heterocyclus; $R^{11}$ = H, (subst.) Alkyl, Cycloalkyl, (subst.) Alkenyl, Cycloalkenyl, Alkinyl, (subst.) Phenyl, Furfuryl, Tetrahydrofurfuryl oder ein Kationäquivalent einer Base; $R^{12}$ = Alkyl, (subst.) Phenylalkyl, Alkenyl oder (subst.) Phenyl; $R^8$ und $R^9$ = Alkanoyl, Benzoyl, Alkoxycarbonyl oder Cyano bedeuten, sowie deren pflanzen-verträgliche Metallsalzkomlexe, Säureadditionsverbindungen und Quaternierungsprodukte besitzen vorteilhafte fungizide und pflanzenwachstumsregulatorische Wirkung.

Neue Triazolylalkylderivate, Verfahren zu ihrer Herstellung sowie ihre Verwendung als Pflanzenbehandlungsmittel

Es ist bekannt, daß bestimmte Triazolylalkylthioether (DE-A 31 08 770) fungizide und wuchsregulatorische Eigenschaften besitzen. Ihre fungizide Wirkung ist jedoch, insbesondere bei niedrigen Aufwandmengen und -konzentrationen, nicht voll befriedigend. Auch die pflanzenwachstumsregulierende Wirkung dieser Azolderivate ist nicht ausreichend.

Weiterhin ist bekannt, daß bestimmte Bisazolylverbindungen (DE-A 32 15 360) als Pflanzenwachstumsregulatoren wirken. Die Wirkung dieser Azolderivate ist jedoch ebenfalls, insbesondere bei niedrigen Aufwandmengen und -konzentrationen, unbefriedigend.

Es wurden nun neue Triazolylalkylderivate mit interessanten pflanzenwachstumsregulierenden und fungiziden Eigenschaften gefunden.

Gegenstand der vorliegenden Erfindung sind daher die Verbindungen der Formel (I),

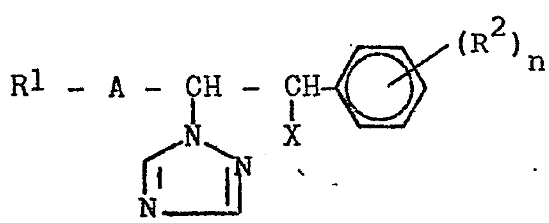

(I)

worin

A eine Carbonylgruppe oder die Gruppierung -CH(OH)-

$R^1$ Phenyl das gegebenenfalls bis zu dreifach durch Halogen, Trifluormethyl, $NO_2$, $(C_1-C_4)$Alkoxy, $(C_1-C_4)$Alkyl und/oder einfach durch Phenyl, Phenoxy oder Halogenphenoxy substituiert ist gegebenenfalls durch $(C_1-C_4)$Alkyl substituiertes Cyclo$(C_3-C_7)$alkyl oder Bicyclo$(C_6-C_{14})$alkyl insbesondere [2.2.1]Bicycloheptyl, oder die Gruppierung $-CHR^3R^4$, in welcher

$R^3$ Wasserstoff oder $(C_1-C_4)$Alkyl, insbesondere Methyl,

$R^4$ Wasserstoff, $(C_1-C_3)$Alkyl, insbesondere Methyl, Halogen$(C_1-C_4)$alkyl, Cyclo$(C_3-C_6)$alkyl, Bicyclo$(C_6-C_{14})$alkyl, insbesondere [2.2.1]-Bicycloheptyl, das durch $(C_1-C_4)$alkyl ein oder mehrfach substituiert sein kann, Phenyl, Halogenphenyl, Phenyl-$(C_1-C_4)$alkyl, Halogenphenyl-$(C_1-C_4)$alkyl oder $(C_1-C_4)$Alkoxy-carbonyl-$(C_1-C_4)$alkyl,

$R^2$ Halogen, Halogen$(C_1-C_4)$alkyl oder Halogen$(C_1-C_4)$-alkoxy mit jeweils bis zu 5 Halogenen, insbesondere Fluor oder Chlor, $(C_1-C_6)$Alkyl, Cyclo$(C_5-C_7)$alkyl, $(C_1-C_6)$Alkoxy, $(C_1-C_6)$-Alkylthio, $(C_2-C_6)$Alkenyloxy, Di$(C_1-C_6)$alkylamino, Nitro, Cyano, Hydroxy, $(C_1-C_4)$Alkoxycarbonyl, Phenyl, Phenoxy oder im Falle n = 2 können zwei Reste $R^2$ zusammen eine gegebenenfalls ungesättigte Kohlenwasserstoffkette mit 3 bis 4 C-Atomen bilden, die benachbarte Ringpositionen miteinander verbinden,

n 0, 1, 2 oder 3, wobei bei Mehrfachsubstitution die Reste $R_2$ verschiedene Bedeutungen besitzen können,

X $-S(O)_mR^5$; $-NR^6R^7$, $CHR^8R^9$,

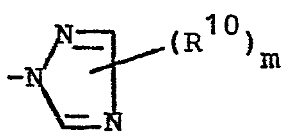
,
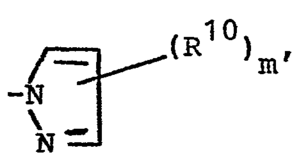
,
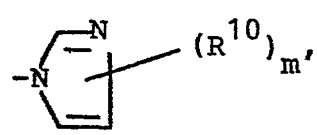
,

wobei

m 0, 1 oder 2

$R^5$ $(C_1-C_{12})$Alkyl, das gegebenenfalls bis zu dreifach durch Hydroxy, Halogen oder $(C_1-C_4)$Alkoxy substituiert ist, $(C_2-C_6)$Alkenyl, $(C_2-C_6)$Alkinyl, Cyclo-$(C_3-C_7)$alkyl, Amino$(C_1-C_6)$alkyl, Di$(C_1-C_6)$alkylamino-$(C_1-C_6)$alkyl, Dicarboxy-$(C_1-C_4)$alkyl, $R^{11}$-O-CO-$(C_1-C_4)$alkyl, das im $(C_1-C_4)$Alkylteil durch Amino substituiert sein kann, $R^{12}$-S-CO-$(C_1-C_4)$alkyl, $R^6R^7$N-CO-$(C_1-C_4)$alkyl, $(C_1-C_8)$-Alkanoyl, Benzoyl, Phenyl oder Benzyl, wobei die drei letztgenannten Reste gegebenenfalls ein oder zweifach durch Halogen oder eine Carboxygruppe substituiert sein können, Furfuryl oder einen fünf- oder sechsgliedrigen, gegebenenfalls benzokondensierten Ring mit 1-3 Heteroatomen aus der Gruppe O, S und N, bevorzugt Pyridyl, wobei der Heterocyclus durch Halogen substituiert sein kann mit der Maßgabe, daß für $R^5$ = $(C_1-C_8)$Alkanoyl oder Benzoyl m = 0 sein muß,

$R^6$ Wasserstoff, $(C_1-C_8)$Alkyl, gegebenenfalls durch Halogen substituiertes Phenyl,

$R^7$ Wasserstoff, $(C_1-C_8)$Alkyl, das gegebenenfalls bis zu dreifach durch Phenyl, Hydroxy, $(C_1-C_6)$Alkoxy, Cyano, Carboxy, $(C_1-C_4)$Alkoxy-carbonyl substituiert ist, Amino, Di$(C_1-C_4)$alkylamino, $(C_1-C_4)$-Alkyl[phenyl-$(C_1-C_2)$alkyl]-amino, $(C_1-C_8)$-Alkylidenamino, Cyclo$(C_5-C_{12})$alkylidenamino, Benzylidenamino, wobei der letztgenannte Rest im Phenylkern durch Halogen, Cyano oder Phenoxy substituiert sein kann, oder

$R^6$ und $R^7$

zusammen mit dem N-Atom, das die beiden Reste

trägt, einen Piperidinring oder Morpholinring, die gegebenenfalls durch $(C_1-C_4)$Alkyl substituiert sein können, insbesondere 3,5-Dimethylpiperidinyl und 3,5-Dimethylmorpholinyl, sowie einen Piperazinring, der in Position 4 durch $(C_2-C_4)$Alkanoyl, $(C_1-C_4)$Alkyl, Phenyl oder Benzyl substituiert ist, wobei die beiden letztgenannten Reste durch Halogen oder $CF_3$ substituiert sein können,

$R^8$, $R^9$ unabhängig voneinander $(C_1-C_3)$Alkanoyl, Benzoyl, $(C_1-C_3)$Alkoxycarbonyl oder Cyano,

$R^{10}$ $(C_1-C_4)$Alkyl,

$R^{11}$ H, $(C_1-C_{18})$Alkyl, das gegebenenfalls bis zu dreifach durch Halogen, vorzugsweise Fluor, Chlor, oder Brom, Hydroxy, $(C_1-C_6)$Alkoxy, $(C_1-C_4)$Alkylthio, $(C_1-C_6)$Alkoxy-$(C_2-C_6)$alkoxy, $(C_1-C_4)$Alkylamino, Di$(C_1-C_4)$alkylamino, Phenyl oder $(C_1-C_4)$Alkoxycarbonyl substituiert ist, Cyclo$(C_3-C_7)$alkyl, $(C_3-C_6)$Alkenyl, Halogen$(C_3-C_6)$alkenyl, Cyclo-$(C_5-C_6)$alkenyl, $(C_3-C_4)$Alkinyl, das gegebenenfalls ein- oder zweifach durch $(C_1-C_6)$-Alkyl, Phenyl, Halogen oder $(C_1-C_2)$Alkoxy substituiert ist, Phenyl, das gegebenenfalls ein- bis dreifach durch $(C_1-C_4)$Alkyl, $(C_1-C_4)$Alkoxy, Halogen, $NO_2$ oder $CF_3$ substituiert ist, Furfuryl, Tetrahydrofurfuryl oder ein Kationäquivalent einer organischen oder anorganischen Base, insbesondere $NH_4$, Mono-, Di-, Tri$(C_1-C_4)$alkylammonium, Benzyltri$(C_1-C_4)$alkylammonium, Natrium oder Kalium,

$R^{12}$ $(C_1-C_6)$Alkyl, Phenyl-$(C_1-C_2)$alkyl, wobei der Phenylrest ein- oder zweifach durch $(C_1-C_4)$Alkyl oder Halogen substituiert sein kann, $(C_3-C_6)$-Alkenyl oder Phenyl, das ein- oder zweifach durch $(C_1-C_4)$Alkyl oder Halogen substituiert sein kann,

bedeuten, sowie deren pflanzenverträgliche Metallsalzkomplexe, Säureadditionsverbindungen und Quaternisierungsprodukte.

Die Reste Halogenphenoxy, Halogenphenyl, Halogenphenylalkyl enthalten bevorzugt 1 bis drei Halogenatome, insbesondere Flur oder Clor.

Bevorzugt von den Definitionen für den Rest $R^2$ sind Halogen, insbesondere Fluor und Chlor, Halogen($C_1$-$C_4$)-alkyl, insbesondere $CF_3$, Halogen($C_1$-$C_4$)alkoxy, insbesondere fluoriertes Alkoxy, besonders bevorzugt $OCF_2CF_2H$ und $OCF_3$, und ($C_1$-$C_4$)Alkoxy-carbonyl, insbesondere $-CO_2CH_3$.

Weiterhin werden solche Verbindungen der Formel (I) bevorzugt, bei denen X für $SR^5$, $NR^6R^7$ oder Triazol steht.

Die Verbindungen der Formel (I) besitzen zwei asymmetrische Kohlenstoffzentren; sie können deshalb in Form ihrer reinen Stereoisomeren oder als Stereoisomerengemische vorliegen; diese werden alle von vorliegender Erfindung erfaßt.

Gegenstand der vorliegenden Erfindung ist ferner ein Verfahren zur Herstellung der Verbindungen der Formel (I), sowie deren Metallsalzkomplexe, Säureadditionsverbindungen und Quaternisierungsprodukte, bei dem man eine Verbindung der Formel (II)

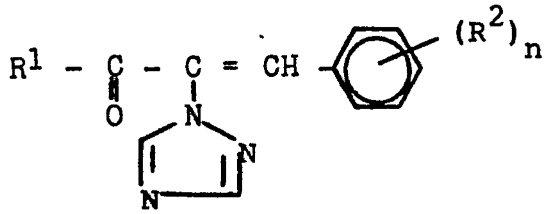

(II)

mit einer Verbindung der Formel (III)

$$H - X'$$

(III)

worin X' die für X genannten Reste mit Ausnahme von $-SOR^5$ und $SO_2R^5$ bedeutet, umsetzt, die erhaltenen Verbindungen der Formel (I), worin A eine Carbonylgruppe bedeutet, gegebenenfalls zu Verbindungen der Formel (I), worin A die Gruppe -CH(OH)- bedeutet, reduziert, die erhaltenen Verbindungen der Formel (I) im Falle X = $SR^5$ gegebenenfalls zu Verbindungen der Formel (I) mit X = $SOR^5$ oder $SO_2R^5$ oxidiert und die Verbindungen der Formel (I) gegebenenfalls in ein Säureadditionssalz, Komplexsalz oder Quaternisierungsprodukt überführt.

Die Vinyltriazol-Derivate der Formel (II) sind teilweise bekannt (vgl. JP-A 53 130 661; DE-A 28 38 847, DE-A 30 10 560, DE-A 29 29 602 und DE-A 30 00 643). Sie können nach den dort angegebenen Verfahren erhalten werden, in dem man beispielsweise Ketone mit Benzaldehyden in üblicher Weise in Gegenwart eines inerten organischen Lösungsmittels, wie beispielsweise Toluol, und in Gegenwart eines Katalysators, wie beispielsweise Piperidinacetat, bei Temperaturen zwischen 20° und 160°C, insbesondere bei der Siedetemperatur des jeweiligen Lösungsmittels, umsetzt.

Die Vinyltriazol-Derivate der Formel (II) kommen in den geometrischen Isomeren E (trans) und Z (cis) vor. Sie können sowohl als E/Z-Isomerengemisch als auch in Form der reinen Isomeren eingesetzt werden.

Die Verbindungen der Formel (III) sind allgemein bekannt; von diesen seien beispielsweise genannt:
Ethylmercaptan, Methylmercaptan, 2-Aminoethylmercaptan, Thioglykolsäure, Thioäpfelsäure, Cystein, Thioessigsäure, Thiobenzoesäure, 2-Hydroxy-ethylmercaptan, 2,3-Dihydroxypropylmercaptan, Thiophenol, 4-Chlorbenzylmercaptan, Thiosalicylsäure, Furfurylmercaptan, 2-Mercaptobenzthiazol, 6-Chlor-2-mercaptobenzoxazol, 2-Mercaptotriazol, 2-Mercaptopyridin, 2-Mercaptopyrimidin, 2-Mercaptothiazolin, Propylamin, Cyanmethylamin, 2-Phenylethylamin, 2-Methoxy-ethylamin, Methylhydrazin, Dimethylhydrazin, Cyclohexylidenmethylhydrazin, Piperidin, Morpholin, 1-Acetyl-piperazin, 1-(3-Trifluormethylphenyl)-piperazin, 1-Benzylipiperazin, Acetylaceton, Dibenzoylmethan, Benzoylaceton, Malonsäurediethylester, Cyanessigsäureethylester.

Die Umsetzungen der Verbindungen der Formel (II) mit Verbindungen der Formel (III) werden in der Regel in Gegenwart eines inerten organischen Lösungsmittels durchgeführt. Geeignet sind beispielsweise Ketone, vorzugsweise Aceton und Methylethylketon, Alkohole, vorzugsweise Methanol, Ethanol und Isopropanol, Nitrile, vorzugsweise Acetonitril und Propionitril, aromatische Kohlenwasserstoffe, vorzugsweise Benzol, Toluol oder Xylol sowie Ether, vorzugsweise Diethylether oder Tetrahydrofuran.

Ferner kann bei dieser Reaktion als Katalysator eine Base zugesetzt werden. Hierfür kommen in Frage: Alkalimetallakoholate, wie Natrium- oder Kaliummethylat oder -ethylat, Alkalimetallamide, wie Natrium- oder Kaliumamid, Alkalimetallhydride, wie Natriumhydrid sowie vorzugsweise tertiäre organische Amine, wie Trialkylamine, insbesondere Triethylamin, Pyridin oder Diazabicyclooctan.

Die Reaktionstemperaturen können in einem größeren Bereich variiert werden. Im allgemeinen arbeitet man zwischen 0° und 120°C, vorzugsweise bei 20° bis 80°C.

Für die Reduktion der Verbindungen der Formel (I), worin A eine Carbonylgruppe bedeutet, eignen sich als Reduktionsmittel bevorzugt komplexe Hydride, insbesondere Natriumborhydrid. Man arbeitet in Gegenwart eines Lösungsmittels, insbesondere eines Alkohols, vorzugsweise Methanol, bei Temperaturen zwischen 0° und 100°C, vorzugsweise 25° bis 60°C.

Die Oxidation der Verbindungen der Formel (I) mit X = $SR^5$ erfolgt mit üblichen Oxidationsmitteln wie $H_2O_2$, Peressigsäure, m-Chlorperbenzoesäure, Perjodsäure, Chromsäue, Kaliumpermanganat nach bekannten üblichen Methoden.

Die neuen Azolylalkylderivate sind als basische Verbindungen zur Bildung von Salzen, Komplexsalzen und Quaternisierungsprodukten befähigt. Genannt seien Salze mit organischen und anorganischen Säuren, wie Acetate, Fumarate, Oxalate, Benzoate, Phenolate, Nitrate, Bromide, Chloride, Sulfate, Sulfonate, Komplexe mit Metallen der Gruppe Ib, IIb, IVb oder VIII des Periodensystems, insbesondere mit Kupfer, Zink, Zinn und Nickel sowie Quaternisierungsprodukte mit ($C_1$-$C_6$)Alkyl- und Phenacylhalogeniden. Die Herstellung derartiger Derivate erfolgt nach allgemein üblichen Methoden.

Die erfindungsgemäßen Verbindungen besitzen intensive pflanzenwachstumsregulierende und mikrobiozide Wirkungen.

So zeichnen sie sich durch hervorragende fungizide Wirksamkeit aus. Selbst in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der neuen Verbindungen erfaßt ferner eine Vielzahl verschiedener phytopathogener Pilze, wie z.B. Piricularia oryzae, Plasmopara viticola, verschiedene Rostarten, Pellicularia sasakii, Venturia inaequalis und Cercospora beticola, vor allem aber echte Mehltaupilze im Obst-, Gemüse-, Getreide- und Zierpflanzenbau.

Die Verbindungen der Formel (I) eignen sich ferner für den Einsatz in technischen Bereichen, beisielsweise als Holzschutzmittel, als Konservierungsmittel in Anstrichfarben, in Kühlschmiermitteln für die Metallbearbeitung oder als Konservierungsmittel in Bohr- und Schneideölen.

Die erfindungsgemäßen Substanzen weisen außerdem hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Des weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann. Besonders hervorzuheben ist die wachstumsregulatorische Wirksamkeit der Verbindungen als Wuchshemmer in Getreide, Mais, Soja, Baumwolle, Rasen sowie ihre Fähigkeiten, den Gehalt an erwünschten Inhaltsstoffen wie Kohlehydraten und Protein bei Nutzpflanzen zu erhöhen. Schließlich zeigen die Verbindungen eine sehr gute Verbesserung der Fruchtabszission, insbesondere bei Zitrusfrüchten, oder eine Reduktion der Haltekraft.

Gegenstand der vorliegenden Erfindung sind daher auch pflanzenwachstumsregulierende und fungizide Mittel, die die obengenannten Verbindungen der Formel (I) oder deren Metallsalzkomplexe, Säureadditionsverbindungen und Quaternisierungsprodukte enthalten.

Die Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Unter Spritzpulvern werden in Wasser gleichmäßig dispergierbare Präparate verstanden, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Ihre Herstellung erfolgt in üblicher Weise z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil ganz oder auch teilweise entfallen. Als Emulgatoren können beispielsweise verwendet werden: Alkylarylsulfonsäure Calciumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel werden durch Vermahlen des Wirkstoffes mit fein verteilten, festen Stoffen, z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen vn Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration etwa 10 bis 90 Gew.-%; der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 1 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen

Wirkstoffen, wie z.B. Insektiziden Akariziden, Herbiziden, Düngemitteln, oder weiteren Wachstumsregulatoren oder Fungiziden sind gegebenenfalls möglich.

Die benötigten Aufwandmengen der Verbindungen der Formel (I) können je nach Indikation innerhalb weiter Grenzen schwanken und variieren auch in Abhängigkeit von äußeren Bedingungen wie Bodenverhältnissen und Klimabedingungen. Im allgemeinen liegen sie jedoch zwischen 0,01 - 10 kg Wirkstoff/ha. Im Falle der Anwendung als Pflanzenwachstumsregulatoren variieren die Aufwandmengen insbesondere im Bereich zwischen 0,15 und 1,25 kg/ha, im Falle der fungiziden Anwendung insbesondere zwischen 0,15 bis 0,5 kg/ha.

Die folgenden Beispiele dienen der weiteren Erläuterung der Erfindung.

A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 64 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 20 Gew.-Teile Wirkstoff mit 6 Gew.-Teilen Alkylphenolpolyglykolether (z.B. (R)Triton X 207, von Rohm & Haas & Co.), 3 Gew.-Teilen Isotridecanolpolyglykolether (8 AeO) und 71 Gew.-Teilen paraffinischem Mineralöl (Siedebereich z.B. ca.255 bis über 377°C) mischt und in einer Reibkugelmühle auf eine Feinheit von unter 5 Mikron vermahlt.

d) Ein emulgierbares Konzentrat läßt sich herstellen aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

0153657

## B. Herstellungsbeispiele

### Beispiel 1

1-(4-Chlorphenyl)-1-ethylthio-4-methyl-2-(1,2,4-triazol-1-yl)-3-pentanon

Man suspendierte 27,7 g (0,1 mol) 1-(4-Chlorphenyl)-4-methyl-2-(1,2,4-triazol-1-yl)-1-penten-3-on in 60 ml Ethanol, setzte 0,3 ml Triethylamin zu und versetzte mit 6,2 g (0,1 mol) Ethylmercaptan. Es entstand eine klare Lösung, aus der nach kurzer Zeit farblose Kristalle ausfielen. Man rührte 2 h bei Raumtemperatur nach und isolierte durch Absaugen 28,6 g (85 % d.Th.) einer farblosen Substanz vom Schmp. 143-5°C als Diastereomerengemisch.

In analoger Weise wurden die Verbindungen der nachfolgenden Tabelle I hergestellt.

Tabelle I

0153657

| Beispiel | Ausgangsmaterialien | | | | Verfahrensprodukte |
|---|---|---|---|---|---|
| | 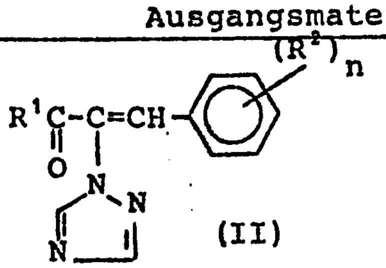 (II) | | HX'(III) | | |
| | $R^1$ | $(R^2)_n$ | X' | A | Smp (°C) |
| 2 | $CH_3$ | 2,4-$Cl_2$ | 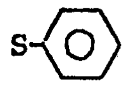 | CO | Oel |
| 3 | " | 2-$CO_2CH_3$ | 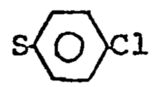 | " | " |
| 4 | " | " | 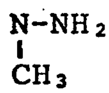 | " | 114-5 |
| 5 | $(CH_3)_2CH$- | 4-Cl | 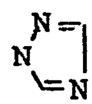 | " | 136-8 |
| 6 | " | " | $SC_8H_{17}$ | " | 93 |
| 7 | " | " | 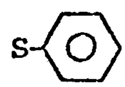 | " | 158 |
| 8 | " | " | 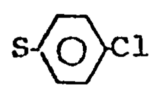 | " | 151-2 |
| 9 | " | " | $SCH_2CO_2CH_3$ | " | 112-3 |
| 10 | " | " | 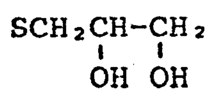 | " | 153-4 |
| 11 | " | " | CH-$(COCH_3)_2$ | " | 165-6 |
| 12 | " | 2,4-$Cl_2$ | " | " | 148 |
| 13 | " | 4-Cl | 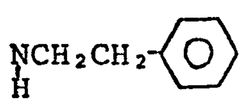 | " | Oel |
| 14 | " | " | 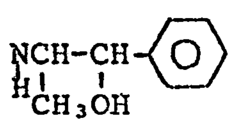 | " | Oel |

...

| Beispiel | $R^1$ | $(R^2)_n$ | X' | A | Smp (°C) |
|---|---|---|---|---|---|
| 15 | $(CH_3)_2CH$ | 4-Cl | 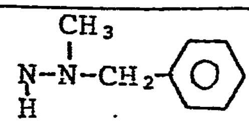 | CO | Oel |
| 16 | " | " | $NHCH_2CH_2OCH_3$ | " | 98-9 |
| 17 | " | " | $NH-CH_2CN$ | " | 141-2 |
| 18 | " | " | 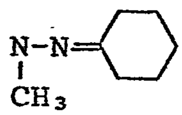 | " | Oel |
| 19 | " | " | $NHC_3H_7$ | " | 94-6 |
| 20 | " | " | $SCH_2CH_2N(C_2H_5)_2$ | " | Oel |
| 21 | " | " | 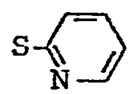 | " | 137-9 |
| 22 | " | $3-OCF_2CF_2H$ | 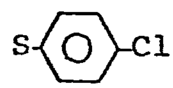 | " | 129-30 |
| 23 | " | $2,4-Cl_2$ | 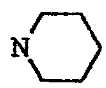 | " | 129-30 |
| 24 | " | " | 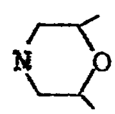 | " | amorph |
| 25 | " | " | 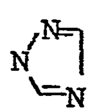 | " | Oel |
| 26 | " | " | $NHN(CH_3)_2$ | " | 142 (Zers.) |
| 27 | " | " | $SCH_2CHOHCH_2OH$ | " | Oel |
| 28 | " | 4-Cl | $SCH(CH_3)COOH$ | " | 128-9 |
| 29 | $i-C_4H_9$ | " | 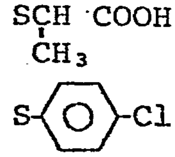 | " | 131-2 |

...

| Beispiel | $R^1$ | $(R^2)_n$ | X' | A | Smp (°C) |
|---|---|---|---|---|---|
| 30 | $i-C_4H_9$ | 4-Cl | $SCH_2CO_2CH_3$ | CO | Oel |
| 31 | " | " | $SCH_2CHOHCH_2OH$ | " | 138 |
| 32 | " | $2,4-Cl_2$ | 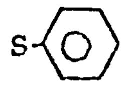 | " | Oel |
| 33 | " | " | $SCH_2CO_2CH_3$ | " | " |
| 34 | " | $2-CO_2CH_3$ | $SCH_2CHOHCH_2OH$ | " | " |
| 35 | " | " | 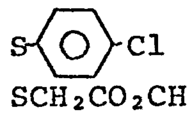 | " | " |
| 36 | 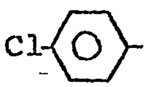 | 4-Cl | $SCH_2CO_2CH_3$ | " | 116-9 |
| 37 | $CH_3$ | $2-CO_2CH_3$ | 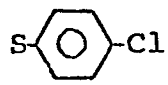 | " | Oel |
| 38 | " | " | 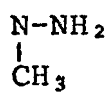 | " | 1145 |
| 39 | " | 4-Cl | $SC_2H_5$ | " | Oel |
| 40 | $i-C_3H_7$ | $2,4-Cl_2$ | $SCH_2CH_2NH_2$ | " | 175-86 |
| 41 | " | " | 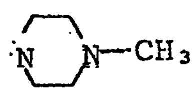 | " | 112-4 |
| 42 | " | 4-Cl | 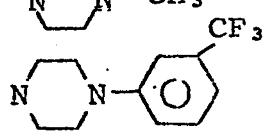 | " | 112-3 |
| 43 | " | " | $SC_2H_5$ | " | 143-5 |
| 44 | " | " | $SC(CH_3)_3$ | " | 183-4 |
| 45 | " | " | $SCOCH_3$ | " | 158-60 |
| 46 | " | " | 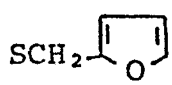 | " | 122-3 |
| 47 | $i-C_3H_7$ | " | 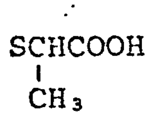 | " | 128-9 |

| Beispiel | $R^1$ | $(R^2)_n$ | X' | A | Smp (°C) |
|---|---|---|---|---|---|
| 48 | $i-C_3H_7$ | 4-Cl | $S-\underset{COOH}{CH}CH_2COOH$ | CO | 196-8 |
| 49 | " | " | $SCH_2\underset{NH_2}{CH}COOH$ | " | 166-7 |
| 50 | " | " | 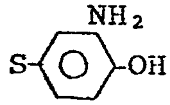 | " | 203-6 |
| 51 | " | $2,4-Cl_2$ | $SC_2H_5$ | " | Oel |
| 52 | $i-C_4H_9$ | " | $S-C(CH_3)_3$ | " | 140-1 |
| 53 | 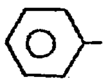 | H | 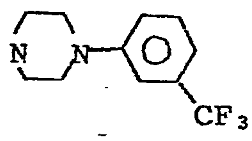 | " | 137-8 |
| 54 | " | 4-Cl | $SCOCH_3$ | " | 174-6 |
| 55 | " | " | SCO-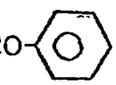 | " | 130-2 |
| 56 | " | " | $SCH(CH_3)_2$ | " | 157-9 |
| 57 | " | " | 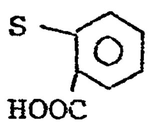 | " | 168-70 |
| 58 | " | " | $SC_2H_5$ | " | 147-8 |
| 59 | " | $2,4-Cl_2$ | " | " | 133-4 |
| 60 | Cl | 4-Cl | $S-\underset{COOH}{CH}-CH_2COOH$ | " | 201-2 |
| 61 | " | $2-CO_2CH_3$ | $SCH_2\underset{OH}{CH}\underset{OH}{CH_2}$ | " | Oel |
| 62 | " | " | $\underset{H}{N}N(CH_3)_2$ | " | " |
| 63 | $i-C_3H_7$ | 4-Cl | $SC_2H_5$ | CH(OH) | " |
| 64 | 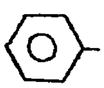 | $2,4-Cl_2$ | " | " | " |

| Beispiel | $R^1$ | $(R^2)_n$ | X' | A | Smp. °C |
|---|---|---|---|---|---|
| 65 | $CH_3$ | 4-Cl | $SCH_2CO_2CH_3$ | CO | Öl |
| 66 | " | " | $SCH_2CO_2C_3H_7$ (i) | " | Öl |
| 67 | " | " | $SCH_2CO_2CH_2CH(C_2H_5)_2$ | " | Öl |
| 68 | " | " | $SCH_2CH_2OH$ | " | Öl |
| 69 | " | " | $SCH_2CH(OH)CH_2OH$ | " | Öl |
| 70 | " | " | $NH-C_3H_7$ | " | Öl |
| 71 | $C_2H_5$ | " | $SCH_2CO_2CH_3$ | " | Öl |
| 72 | " | " | $SCH_2CO_2C_6H_{13}$ | " | Öl |
| 73 | " | " | $SCH(CH_3)CO_2CH_3$ | " | Öl |
| 74 | " | " | $SCH_2CH_2OH$ | " | Öl |
| 75 | " | " | $SCH_2CH(OH)CH_2OH$ | " | Öl |
| 76 | " | " | $S-C_6H_4-4-Cl$ | " | 137-8 |
| 77 | $(CH_3)_2CH$ | " | $SCH_2CO_2C_2H_5$ | " | 103-5 |
| 78 | " | " | $SCH_2CO_2C_3H_7$ (i) | " | 99-102 |
| 79 | " | " | $SCH_2CO_2-C_6H_{11}$ (cyclo) | " | 105 |
| 80 | " | " | $SCH_2CO_2C_6H_{13}$ | " | 97-9 |
| 81 | " | " | $SCH_2CO_2C_{12}H_{25}$ | " | 44-6 |
| 82 | " | " | $SCH_2CO_2CH_2CH(C_2H_5)_2$ | " | 65-70 |
| 83 | " | " | " | CH(OH) | Öl |
| 84 | " | " | $SCH(CH_3)CO_2CH_3$ | CO | 76-80 |
| 85 | " | " | $SCH(CH_3)CO_2C_2H_5$ | " | Öl |
| 86 | " | " | $SCH(CH_3)CO_2$ $C_3H_7$ (i) | " | Öl |
| 87 | " | " | $SCH(CH_3)CO_2CH_2CH_2OCH_3$ | " | Öl |
| 88 | " | " | $SCH_2CO_2CH_2CH_2OCH_3$ | " | Öl |
| 89 | " | 4-Cl | $SCH_2CH_2OH$ | " | 139-40 |
| 90 | " | " | $S-C_6H_{11}$ (cyclo) | " | 111-3 |
| 91 | " | " | $S-CH(CH_3)C_2H_5$ | " | 108-13 |
| 92 | " | 4-F | $S-C_6H_4-4-Cl$ | " | 149-50 |

| Beispiel | $R^1$ | $(R^2)_n$ | X' | A | Smp. °C |
|---|---|---|---|---|---|
| 93 | $(CH_3)_2CH$ | 4-F | $SCH_2CO_2CH_3$ | CO | 101-2 |
| 94 | " | " | $SCH_2CH(OH)CH_2OH$ | " | 116-7 |
| 95 | " | $4-OC_2H_5$ | $S\ CH_2CO_2CH_3$ | " | Öl |
| 96 | " | " | $S-C_6H_4-4-Cl$ | " | 92-3 |
| 97 | " | $4-CO_2CH_3$ | " | " | 142-4 |
| 98 | " | " | $SCH(CH_3)CO_2C_2H_5$ | " | Öl |
| 99 | " | 3-Br | $S-C_6H_4-4-Cl$ | " | 132-4 |
| 100 | " | " | $SCH_2CO_2CH_3$ | " | Öl |
| 101 | " | " | $SCH(CH_3)CO_2CH_2CH_2OCH_3$ | " | Öl |
| 102 | " | " | $SCH_2CH(OH)CH_2OH$ | " | Öl |
| 103 | " | 2-Cl | " | " | Öl |
| 104 | " | " | $SCH_2CO_2CH_3$ | " | Öl |
| 105 | " | " | $SCH(CH_3)CO_2C_3H_7$ (i) | " | Öl |
| 106 | " | $2-OCH_3$ | $S-C_6H_4-4-Cl$ | " | 59-61 |
| 107 | " | " | $SCH_2CO_2CH_3$ | " | Öl |
| 108 | " | " | $SCH_2CO_2\ C_6H_{13}$ | " | Öl |
| 109 | " | " | $SCH_2CH(OH)CH_2OH$ | " | Öl |
| 110 | " | H | $S-C_6H_4-4-Cl$ | " | 130-2 |
| 111 | " | " | $SCH_2CO_2CH_3$ | " | 113-5 |
| 112 | " | " | $SCH(CH_3)CO_2C_3H_7$ (i) | " | Öl |
| 113 | " | " | $SCH_2CH_2OH$ | " | 144-6 |
| 114 | $(CH_3)_2CHCH_2$ | 3-Cl | $SCH_2CO_2CH_3$ | " | Öl |
| 115 | " | " | $SCH_2CH(OH)CH_2OH$ | " | Öl |
| 116 | 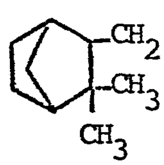 | 4-Cl | $S-C_6H_4-4-Cl$ | " | Öl |
| 117 | " | " | $SCH_2CO_2CH_3$ | " | Öl |
| 118 | $CH_3O\underset{\overset{\|}{O}}{C}CH_2CH_2$ | " | $S-C_6H_4-4-Cl$ | " | 87-8 |
| 119 | " | " | $SCH_2CO_2CH_3$ | " | Öl |

| Beispiel | $R^1$ | $(R^2)_n$ | X' | A | Smp.°C |
|---|---|---|---|---|---|
| 120 | 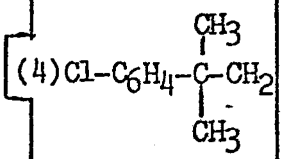 | " | $SCH_2CO_2CH_3$ | " | Öl |
| 121 | " | " | $SCH_2CH_2OH$ | " | 132-4 |
| 122 | $C_6H_5$ | " | " | " | 146-7 |
| 123 | " | " | $NH-N(CH_3)_2$ | " | Öl |
| 124 | " | " | $SCH_2CO_2CH_3$ | " | 109-11 |
| 125 | " | " | $SCH_2CO_2C_2H_5$ | " | 106-8 |
| 126 | " | " | $SCH_2CO_2C_6H_{13}$ | " | Öl |
| 127 | " | " | $S-C_6H_5$ | " | 149-51 |
| 129 | " | " | " | CH(OH) | 164-7 |

## C. Biologische Beispiele

### Beispiel 1

Weizenpflanzen wurden im 3-Blattstadium mit Konidien des Weizenmehltaus (Erysiphe graminis) stark inokuliert und in einem Gewächshaus bei 20 °C und einer relativen Luftfeuchte von 90 - 95 % aufgestellt. 3 Tage nach Inokulation wurden die Pflanzen mit den zu prüfenden Verbindungen in den Wirkstoffkonzentrationen von 500, 250 und 125 mg/Liter Spritzbrühe tropfnaß gespritzt. Nach einer Inkubationszeit von 10 Tagen untersuchte man die Pflanzen auf Befall mit Weizenmehltau.

Der Befallsgrad wird ausgedrückt in % befallener Blattfläche, bezogen auf unbehandelte, infizierte Kontrollpflanzen (= 100 % Befall). Die Ergebnisse zeigt die nachstehende Tabelle II.

Tabelle II

| Verb. gem. Beispiel | mit Weizenmehltau befallene Blattfläche in % bei mg ... Wirkstoff/Liter Spritzbrühe | | |
|---|---|---|---|
| | 500 | 250 | 125 |
| 2 | 0 | 0 | 0 |
| 3 | 0 | 0 | 0 |
| 5 | 0 | 0 | 0 |
| 6 | 0 | 0 | 0 |
| 9 | 0 | 0 | 0 |
| 10 | 0 | 0 | 0 |
| 11 | 0 | 0 | 0 |
| 12 | 0 | 0 | 0 |
| 15 | 0 | 0 | 0 |
| 16 | 0 | 0 | 0 |
| 18 | 0 | 0 | 0 |
| 19 | 0 | 0 | 0 |

Fortsetzung Tabelle II

| Verb. gem. Beispiel | mit Weizenmehltau befallene Blattfläche in % bei ... mg Wirkstoff/Liter Spritzbrühe 500 | 250 | 125 |
|---|---|---|---|
| 20 | 0 | 0 | 0 |
| 21 | 0 | 0 | 0 |
| 23 | 0 | 0 | 0 |
| 24 | 0 | 0 | 0 |
| 25 | 0 | 0 | 0 |
| 26 | 0 | 0 | 0 |
| 27 | 0 | 0 | 0 |
| 30 | 0 | 0 | 0 |
| 31 | 0 | 0 | 0 |
| 32 | 0 | 0 | 0 |
| unbehandelte, infizierte Pflanzen | | 100 | |

Beispiel 2

Gerstenpflanzen wurden im 3-Blattstadium mit Konidien des Gerstenmehltaus (Erysiphe graminis sp. hordei) stark inokuliert und in einem Gewächshaus bei 20 °C und einer relativen Luftfeuchte von 90 bis 95 % aufgestellt. 3 Tage nach Inokulation wurden die Pflanzen mit den zu prüfenden Verbindungen in den Wirkstoffkonzentrationen von 500, 250 und 125 mg/Liter Spritzbrühe tropfnaß gespritzt. Nach einer Inkubationszeit von 10 Tagen wurde der Befall mit Gerstenmehltau untersucht. Der Befallsgrad wird ausgedrückt in % befallener Blattfläche, bezogen auf unbehandelte, infizierte Kontrollpflanzen (= 100 % Befall). Die Ergebnisse zeigt Tabelle III.

...

0153657

Tabelle III

| Verbindung gemäß Beispiel | mit Gerstenmehltau befallene Blattfläche in % bei ... mg Wirkstoff/Liter Spritzbrühe 500 | 250 | 125 |
|---|---|---|---|
| 5 | 0 | 0 | 0 - 3 |
| 7 | 0 | 0 | 0 |
| 11 | 0 | 0 | 0 |
| 12 | 0 | 0 | 0 |
| 15 | 0 | 0 | 0 |
| 18 | 0 | 0 | 0 |
| 19 | 0 | 0 | 0 |
| 20 | 0 | 0 | 0 |
| 21 | 0 | 0 | 0 |
| 23 | 0 | 0 | 0 |
| 24 | 0 | 0 | 0 |
| 25 | 0 | 0 | 0 |
| 26 | 0 | 0 | 0 |
| 27 | 0 | 0 | 0 |
| 30 | 0 | 0 | 0 |
| 31 | 0 | 0 | 0 |
| 32 | 0 | 0 | 0 |
| 33 | 0 | 0 | 0 |
| 34 | 0 | 0 | 0 |
| unbehandelte, infizierte Pflanzen | | 100 | |

...

0153657

Beispiel 3

Gurkenpflanzen (Sorte Delikateß) wurden im 2-Blattstadium mit einer Konidiensuspension von Gurkenmehltau (Erysiphe cichoracearum) stark inokuliert. Nach einer Antrocknungszeit der Sporensuspension von 30 Minuten wurden die Pflanzen in einem Gewächshaus bei 22 °C und 90 % relativer Luftfeuchte aufgestellt. 3 Tage nach Infektion wurde mit den zu prüfenden Verbindungen in den in Tabelle IV angegebenen Wirkstoffkonzentrationen tropfnaß gespritzt. Nach 10 Tagen erfolgte die Bonitur. Der Befallsgrad wird ausgedrückt in % befallener Blattfläche, bezogen auf unbehandelte, infizierte Kontrollpflanzen (= 100 % Befall). Tabelle IV zeigt die Ergebnisse.

Tabelle IV

| Verbindung gemäß Beispiel | mit Gurkenmehltau befallene Blattfläche in % bei ... mg Wirkstoff/Liter Spritzbrühe | | |
|---|---|---|---|
| | 500 | 250 | 125 |
| 5 | 0 | 0 | 0 |
| 6 | 0 | 0 | 0 |
| 10 | 0 | 0 | 0 |
| 12 | 0 | 0 | 0 |
| 15 | 0 | 0 | 0 |
| 18 | 0 | 0 | 0 |
| 19 | 0 | 0 | 0 |
| 20 | 0 | 0 | 0 |
| 21 | 0 | 0 | 0 |
| 23 | 0 | 0 | 0 |
| 25 | 0 | 0 | 0 |
| 26 | 0 | 0 | 0 |
| 27 | 0 | 0 | 0 |
| 29 | 0 | 0 | 0 |

...

Fortsetzung Tabelle IV

| Verbindung gemäß Beispiel | mit Gurkenmehltau befallene Blattfläche in % bei ... mg Wirkstoff/Liter Spritzbrühe 500 | 250 | 125 |
|---|---|---|---|
| 31 | 0 | 0 | 0 |
| 32 | 0 | 0 | 0 |
| 33 | 0 | 0 | 0 |
| 34 | 0 | 0 | 0 |
| 35 | 0 | 0 | 0 |
| unbehandelte, infizierte Pflanzen | | 100 | |

Beispiel 4

Wuchshemmung an Getreide

In Schalenversuchen im Gewächshaus wurden junge Getreidepflanzen (Weizen, Gerste und Roggen) im 3-Blattstadium mit den in Tabelle I genannten Verbindungen in den angegebenen Wirkstoffkonzentrationen (kg/ha) tropfnaß gespritzt.

Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet. Die Ergebnisse sind in der Tabelle V zusammengefaßt. Bei der Angabe der Wuchshemmung bedeuten 100 % den Stillstand des Wachstums und 0 % ein Wachstum entsprechend dem der unbehandelten Kontrollpflanzen.

...

Tabelle V

| Verbindung gem. Bsp. | Anwendungskonz. (kg/ha) | Wuchshemmung in % Weizen | Gerste | Roggen | phytotox. Wirkung |
|---|---|---|---|---|---|
| 5 | 2,5<br>1,25 | 21<br>21 | 24<br>24 | 40<br>40 | keine Schäden |
| 7 | 2,5<br>1,25 | 18<br>16 | 22<br>22 | 36<br>26 | keine Schäden |
| 8 | 2,5<br>1,25 | 19<br>12 | 30<br>13 | 39<br>36 | keine Schäden |
| 11 | 2,5<br>1,25 | 25<br>24 | 23<br>22 | 37<br>35 | keine Schäden |
| 18 | 2,5<br>1,25 | 19<br>17 | 19<br>16 | 29<br>27 | keine Schäden |
| 19 | 2,5<br>1,25 | 24<br>23 | 23<br>22 | 34<br>34 | keine Schäden |

## Beispiel 5

Wuchshemmung in Wasserreis

Reispflanzen wurden in Kleinparzellen (2 m x 2 m) angezogen und im Stadium der maximalen Bestockung mit den angegebenen Verbindungen behandelt. Die Substanzen können sowohl durch Spritzung appliziert als auch in das Wasser gegeben werden.

3 Wochen nach Behandlung wurde bei allen Pflanzen der Zuwachs gemessen und die Wuchshemmung in % des Zuwachses der Kontrollpflanzen berechnet. Es wurde außer-

...

demauf eine mögliche phytotoxische Wirkung der Verbindungen geachtet.

Die Ergebnisse sind in Tabelle VI zusammengefaßt. Bei der Angabe der Wuchshemmung bedeuten 100 % den Stillstand des Wachstums und 0 % ein Wachstum entsprechend der Kontrolle.

Tabelle VI

| Verbindung gem. Bsp. | Anwendungskonz. (kg/ha) | Wuchshemmung (%) | Phytotox. Wirkung |
|---|---|---|---|
| 5 | 1,25<br>0,62 | 22<br>19 | keine Schäden |
| 7 | 1,25<br>0,62 | 20<br>17 | keine Schäden |
| 8 | 1,25<br>0,62 | 21<br>17 | keine Schäden |
| 11 | 1,25<br>0,62 | 22<br>16 | keine Schäden |
| 18 | 1,25<br>0,62 | 21<br>18 | keine Schäden |
| 19 | 1,25<br>0,62 | 21<br>19 | keine Schäden |

Beispiel 6

Wuchshemmung an Sojabohnen

Ca. 10 cm große Sojabohnen wurden mit den Wirkstoffzubereitungen tropfnaß bespritzt. Nach 3 Wochen wird der Zuwachs gemessen und die Wuchshemmung in % des Zu-

...

wachses der Kontrollpflanzen berechnet. Die Ergebnisse sind in Tabelle VII zusammengefaßt.

Tabelle VII

| Verbindung gem. Bsp. | Anwendungs-konz. (kg/ha) | Wuchshemmung (%) | Phytotox. Wirkung |
|---|---|---|---|
| 5 | 2,5<br>1,25<br>0,62 | 68<br>68<br>52 | keine Schäden |
| 7 | 2,5<br>1,25<br>0,62 | 55<br>55<br>49 | keine Schäden |
| 8 | 2,5<br>1,25<br>0,62 | 65<br>55<br>53 | keine Schäden |
| 19 | 2,5<br>1,25<br>0,62 | 62<br>58<br>56 | keine Schäden |

0153657
HOE 84/F 036

Patentansprüche

1. Verbindungen der Formel (I)

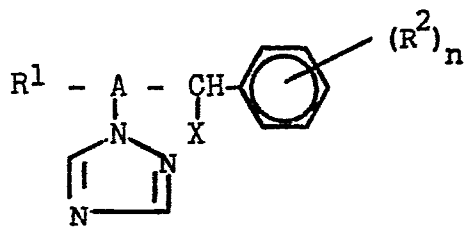

(I)

worin

A eine Carbonylgruppe oder die Gruppierung -CH(OH)-

$R^1$ Phenyl das gegebenenfalls bis zu dreifach durch Halogen, Trifluormethyl, $NO_2$, $(C_1-C_4)$Alkoxy, $(C_1-C_4)$Alkyl und/oder einfach durch Phenyl, Phenoxy oder Halogenphenoxy substituiert ist gegebenenfalls durch $(C_1-C_4)$Alkyl substituiertes Cyclo$(C_3-C_7)$alkyl oder Bicyclo$(C_6-C_{14})$alkyl insbesondere [2.2.1]Bicycloheptyl, oder die Gruppierung $-CHR^3R^4$, in welcher

$R^3$ Wasserstoff oder $(C_1-C_4)$Alkyl, insbesondere Methyl,

$R^4$ Wasserstoff, $(C_1-C_3)$Alkyl, insbesondere Methyl, Halogen$(C_1-C_4)$alkyl, Cyclo$(C_3-C_6)$alkyl, Bicyclo$(C_6-C_{14})$alkyl, insbesondere /2.2.1/-Bicycloheptyl, das durch $(C_1-C_4)$alkyl ein oder mehrfach substituiert sein kann, Phenyl, Halogenphenyl, Phenyl-$(C_1-C_4)$alkyl, Halogenphenyl-$(C_1-C_4)$alkyl oder $(C_1-C_4)$Alkoxy-carbonyl-$(C_1-C_4)$alkyl,

$R^2$ Halogen, Halogen$(C_1-C_4)$alkyl oder Halogen$(C_1-C_4)$-alkoxy mit jeweils bis zu 5 Halogen, insbesondere Fluor oder Chlor, $(C_1-C_6)$Alkyl, Cyclo$(C_5-C_7)$alkyl, $(C_1-C_6)$Alkoxy, $(C_1-C_6)$-

Alkylthio, $(C_2-C_6)$Alkenyloxy, Di$(C_1-C_6)$alkylamino, Nitro, Cyano, Hydroxy, $(C_1-C_4)$Alkoxycarbonyl, Phenyl, Phenoxy oder im Falle n = 2 können zwei Reste $R^2$ zusammen eine gegebenenfalls ungesättigte Kohlenwasserstoffkette mit 3 bis 4 C-Atomen bilden, die benachbarte Ringpositionen miteinander verbinden,

n 0, 1, 2 oder 3, wobei bei Mehrfachsubstitution die Reste $R_2$ verschiedene Bedeutungen besitzen können,

X $-S(O)_mR^5$; $-NR^6R^7$, $CHR^8R^9$,

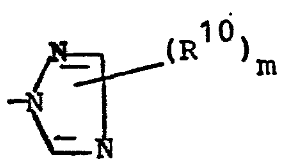 , 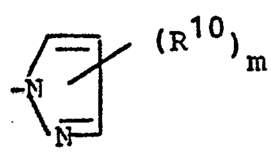 , 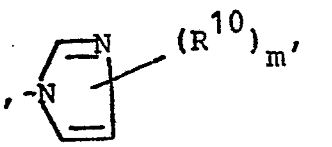

wobei

m 0, 1 oder 2

$R^5$ $(C_1-C_{12})$Alkyl, das gegebenenfalls bis zu dreifach durch Hydroxy, Halogen oder $(C_1-C_4)$Alkoxy substituiert ist, $(C_2-C_6)$Alkenyl, $(C_2-C_6)$Alkinyl, Cyclo-$(C_3-C_7)$alkyl, Amino$(C_1-C_6)$alkyl, Di$(C_1-C_6)$alkylamino-$(C_1-C_6)$alkyl, Dicarboxy-$(C_1-C_4)$alkyl, $R^{11}$-O-CO-$(C_1-C_4)$alkyl, das im $(C_1-C_4)$Alkylteil durch Amino substituiert sein kann, $R^{12}$-S-CO-$(C_1-C_4)$Alkyl, $R^6R^7$N-CO-$(C_1-C_4)$Alkyl, $(C_1-C_8)$-Alkanoyl, Benzoyl, Phenyl oder Benzyl, wobei die drei letztgenannten Reste gegebenenfalls ein oder zweifach durch Halogen oder eine Carboxygruppe substituiert sein können, Furfuryl oder einen fünf- oder sechsgliedrigen, gegebenenfalls benzokondensierten Ring mit 1-3 Heteroatomen aus der Gruppe O, S und N, wobei der Heterocyclus durch Halogen substituiert sein kann, bevorzugt Pyridyl, mit der Maßgabe, daß für $R^5$ = $(C_1-C_8)$Alkanoyl oder Benzoyl m = 0 sein muß,

$R^6$ Wasserstoff, $(C_1-C_8)$Alkyl, gegebenenfalls durch Halogen substituiertes Phenyl,

$R^7$ Wasserstoff, $(C_1-C_8)$Alkyl, das gegebenenfalls bis zu dreifach durch Phenyl, Hydroxy, $(C_1-C_6)$Alkoxy, Cyano, Carboxy, $(C_1-C_4)$Alkoxy-carbonyl substituiert ist, Amino, Di$(C_1-C_4)$alkylamino, $(C_1-C_4)$-Alkyl[phenyl-$(C_1-C_2)$alkyl]-amino, $(C_1-C_8)$-Alkylidenamino, Cyclo$(C_5-C_{12})$alkylidenamino, Benzylidenamino, wobei der letztgenannte Rest im Phenylkern durch Halogen, Cyano oder Phenoxy substituiert sein kann, oder

$R^6$ und $R^7$ zusammen mit dem N-Atom, das die beiden Reste trägt, einen Piperidinring oder Morpholinring, die gegebenenfalls durch $(C_1-C_4)$Alkyl substituiert sein können, insbesondere 3,5-Dimethylpiperidinyl und 3,5-Dimethylmorpholinyl, sowie einen Piperazinring, der in Position 4 durch $(C_2-C_4)$Alkanoyl, $(C_1-C_4)$Alkyl, Phenyl oder Benzyl substituiert ist, wobei die beiden letztgenannten Reste durch Halogen oder $CF_3$ substituiert sein können,

$R^8$, $R^9$ unabhängig voneinander $(C_1-C_3)$Alkanoyl, Benzoyl, $(C_1-C_3)$Alkoxycarbonyl oder Cyano,

$R^{10}$ $(C_1-C_4)$Alkyl,

$R^{11}$ H, $(C_1-C_{18})$Alkyl, das gegebenenfalls bis zu dreifach durch Halogen, vorzugsweise Fluor, Chlor, oder Brom, Hydroxy, $(C_1-C_6)$Alkoxy, $(C_1-C_4)$Alkylthio, $(C_1-C_6)$Alkoxy-$(C_2-C_6)$alkoxy, $(C_1-C_4)$Alkylamino, Di$(C_1-C_4)$alkylamino, Phenyl oder $(C_1-C_4)$Alkoxycarbonyl substituiert ist, Cyclo$(C_3-C_7)$alkyl,

$(C_3-C_6)$Alkenyl, Halogen$(C_3-C_6)$alkenyl, Cyclo-$(C_5-C_6)$alkenyl, $(C_3-C_4)$Alkinyl, das gegebenenfalls ein- oder zweifach durch $(C_1-C_6)$-Alkyl, Phenyl, Halogen oder $(C_1-C_2)$Alkoxy substituiert ist, Phenyl, das gegebenenfalls ein- bis dreifach durch $(C_1-C_4)$Alkyl, $(C_1-C_4)$Alkoxy, Halogen, $NO_2$ oder $CF_3$ substituiert ist, Furfuryl, Tetrahydrofurfuryl oder ein Kationäquivalent einer organischen oder anorganischen Base, insbesondere $NH_4$, Mono-, Di-, Tri$(C_1-C_4)$alkylammonium, Benzyl-tri$(C_1-C_4)$alkylammonium, Natrium oder Kalium,

$R^{12}$ $(C_1-C_6)$Alkyl, Phenyl-$(C_1-C_2)$alkyl, wobei der Phenylrest ein- oder zweifach durch $(C_1-C_4)$Alkyl oder Halogen substituiert sein kann, $(C_3-C_6)$-Alkenyl oder Phenyl, das ein- oder zweifach durch $(C_1-C_4)$Alkyl oder Halogen substituiert sein kann,

bedeuten, sowie deren pflanzenverträgliche Metallsalzkomplexe, Säureadditionsverbindungen und Quaternisierungsprodukte.

2. Verfahren zur Herstellung der Verbindungen gemäß Anspruch 1, dadurch gekennzeichnet, daß man eine Verbindung der Formel (II)

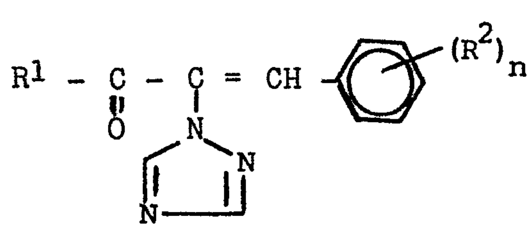

(II)

mit einer Verbindung der Formel (III),

H-X' (III)

worin X' die für X genannten Reste mit Ausnahme von $-SOR^5$ und $-SO_2R^5$ bedeutet, umsetzt, die erhaltenen Verbindungen der Formel (I), worin A eine Carbonylgruppe bedeutet, gegebenenfalls reduziert, die erhaltenen Verbindungen der Formel (I) im Falle X = $SR^5$ gegebenenfalls zu Verbindungen der Formel (I) mit X = $SOR^5$ oder $SO_2R^5$ oxidiert und die Verbindungen der Formel I gegebenenfalls in ein Säureadditionssalz, Komplexsalz oder Quaternisierungsprodukt überführt.

3. Pflanzenwuchsregulierende und fungizide Mittel, gekennzeichnet durch einen wirksamen Gehalt an einer Verbindung gemäß Anspruch 1.

4. Verwendung von Verbindungen gemäß Anspruch 1 als Fungizide oder Wachstumsregulatoren.

5. Verfahren zur Bekämpfung von Schadpilzen, dadurch gekennzeichnet, daß man auf die zu schützenden Pflanzen, Pflanzenteile oder Anbauflächen eine Verbindung gemäß Anspruch 1 aufbringt.

6. Verfahren zur Wachstumsregulierung von Kulturpflanzen, dadurch gekennzeichnet, daß man auf die zu schützenden Pflanzen, Pflanzenteile oder Anbauflächen eine Verbindung gemäß Anspruch 1 aufbringt.

**0153657**
Nummer der Anmeldung

Europäisches Patentamt

# EUROPÄISCHER RECHERCHENBERICHT

EP 85101520.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A2 - 0 092 730 (BASF)<br>* Formel I *<br>-- | 1,3 | C 07 D 249/08<br>C 07 D 401/06<br>C 07 D 401/12<br>C 07 D 403/06<br>C 07 D 405/12<br>A 01 N 43/653 |
| D,A | EP - A1 - 0 059 894 (BAYER)<br>* Zusammenfassung *<br>-- | 1,3 | |
| A | DE - A1 - 2 604 308 (BAYER)<br>* Formel I *<br>-- | 1,4 | |
| A | DE - A1 - 3 151 440 (BAYER)<br>* Anspruch 1 *<br>-- | 1 | |
| A | DE - A1 - 3 139-250 (BASF)<br>* Zusammenfassung *<br>-- | 1,3 | **RECHERCHIERTE SACHGEBIETE (Int. Cl.4)** |
| A | DE - A1 - 3 200 414 (BAYER)<br>* Formel I *<br>-- | 1,3,4 | C 07 D 249/00<br>C 07 D 401/00<br>C 07 D 403/00<br>C 07 D 405/00 |
| A | EP - A1 - 0 068 144 (HOECHST)<br>* Formel I *<br>---- | 1,4 | |

Der vorliegende Recherchenbericht wurde für alle Patentansprüche erstellt.

| Recherchenort | Abschlußdatum der Recherche | Prüfer |
|---|---|---|
| WIEN | 30-04-1985 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82